# EUROPÄISCHE PATENTANMELDUNG

(11) **EP 1 081 134 A1**
(43) Veröffentlichungstag der Anmeldung: **07.03.2001**
(21) Anmeldenummer: 00117381.4
(22) Anmeldetag: 24.08.2000
(51) Int. Cl.: C07C 319/24, C08G 75/04

(54) **Verfahren zur Herstellung von Polythiopolycarbonsäuren**

(30) Priorität: 06.09.1999 DE 19942395
(71) Anmelder: Bayer Aktiengesellschaft, 51368 Leverkusen (DE)
(72) Erfinder: Scholl, Thomas Dr., 51469 Bergisch Gladbach (DE)

(57) **Zusammenfassung**

Polythiopolycarbonsäuren mit einem höheren Gehalt an gebundenem Schwefel können gemäß einem einfachen Verfahren ohne den Erhalt von unerwünschten Abfallprodukten, wie Salzsäure und Kochsalz, in hoher Reinheit hergestellt werden, indem man Mercaptoalkylcarbonsäuren mit Schwefel bei erhöhter Temperatur umsetzt.

## Beschreibung

Die vorliegende Erfindung betrifft ein Verfahren zur Herstellung von Polythiopolycarbonsäuren, welches auf der Umsetzung von Mercaptoalkylcarbonsäuren mit Schwefel bei erhöhter Temperatur beruht. Die so hergestellten Polythiopolycarbonsäuren weisen einen hohen Gehalt an gebundenem Schwefel in Form einer polysulfidischen Verteilung auf und eignen sich beispielsweise zur Herstellung von Kautschukmischungen und Kautschukvulkanisaten.

Polythiodicarbonsäuren der Struktur R¹-(S)ₙ-R² sind aus US-A 5 130 363 bekannt und eignen sich z.B. zur Herstellung von Kautschukvulkanisaten mit verbessertem Hystereseverhalten. Die Herstellung dieser Verbindungen erfolgte hier durch Umsetzung von Mercaptoalkylcarbonsäuren mit Schwefelchloriden, wie Schwefeldichlorid oder Dischwefelchlorid. Nachteilig dabei ist der Umgang mit den korrosiven Schwefelchloriden und dem Nebenprodukt Chlorwasserstoff.

In EP-A 0 780 429 wird ein Herstellungsverfahren beschrieben für eine Mischung aus Di-, Tri- und Tetrathiodipropionsäure ausgehend von 3-Chlorpropionsäure und Natriumpolysulfid in Wasser. Neben der Bildung von Mengen NaCl als Abfallprodukt ist an diesem Verfahren weiter von Nachteil, dass der Gehalt an gebundenem Schwefel eng begrenzt ist, und daher ein Gemisch entsteht, welches zu ca. 70 % aus Dithiodipropionsäure und nur zu ca. 30 % aus Tri- und Tetrathiodipropionsäure besteht.

Aus US-A 4 119 549 ist ein Verfahren zur Herstellung von geschwefelten Verbindungen durch Umsetzung von Olefinen mit Schwefelwasserstoff und Schwefel bekannt. Das Verfahren führt zu uneinheitlichen Prdoukten, wenn als Olefinkomponente solche mit polymerisationsfreudigen Doppelbindungen, insbesondere α,β-ungesättigte Carbonsäuren, eingesetzt werden.

Ziel der vorliegenden Erfindung ist daher ein Herstellungsverfahren für besonders reine Polythiopolycarbonsäuren, das die Herstellung von höheren Gehalten an gebundenen Schwefel gestattet und keine Salzsäure und Kochsalz als unerwünschte Abfallprodukte liefert.

Es wurde jetzt gefunden, dass man Polythiopolycarbonsäuren in hoher Reinheit und mit einem hohen Gehalt an gebundenem Schwefel und ohne Kochsalz oder Chlorwasserstoff als Abfallprodukt auf einfachem Wege durch Erhitzen von Mercaptoalkylcarbonsäuren mit Schwefel enthalten kann.

Gegenstand der vorliegenden Erfindung ist daher ein Verfahren zur Herstellung von Polythiopolycarbonsäuren der Formel

R¹-(S)ₙ-R² (I),

worin
- R¹ und R²: gleich oder verschieden sind und für einen linearen oder verzweigten C₁-C₃₆-Alkylrest, bevorzugt C₁-C₁₂-Alkylrest, oder einen C₅-C₁₈-, bevorzugt C₅-C₁₂-Cycloalkylrest stehen, wobei der Alkylrest und der Cycloalkylrest mit 1 bis 4, bevorzugt 1 bis 2, Carboxyl- oder Carboxylatgruppen substituiert ist und wobei im Falle einer Carboxylatgruppe als Kation eine Ammoniumgruppe, eine C₁-C₁₈-Mono-, Di- oder Trialkylammoniumgruppe, eine C₆-C₁₈-Mono-, Di- oder Triarylammoniumgruppe, eine C₇-C₂₁-Mono-, Di- oder Trialkylarylammoniumgruppe oder ein 1- bis 4-wertiges Metallion vorliegt, und
- n: für eine ganze Zahl von 2 bis 14 steht,
das dadurch gekennzeichnet ist, dass man Mercaptoalkylcarbonsäuren der Formeln

R¹-S-H (IIa),

R²-S-H (IIb),

worin
- R¹ und R²: die obige Bedeutung besitzen,
oder Mischungen entsprechender Mercaptoalkylcarbonsäuren mit Schwefel im Molverhältnis von 1 zu 0,25 bis 5, bevorzugt 1 zu 0,5 bis 3, besonders bevorzugt 1 zu 1 bis 2,5, bei Temperaturen von 70 bis 220°C, bevorzugt 100 bis 170°C, gegebenenfalls in Gegenwart von sauren oder alkalischen Katalysatoren und gegebenenfalls in Anwesenheit eines inerten Lösungsmittels umsetzt.

Als Reste R¹ und R² kommen beispielsweise in Frage:
-CH₂COOH, -CH₂CH₂COOH, -CH(CH₃)COOH, -CH(COOH)CH₂COOH, -Cyclohexyl-COOH.

Als Kationen dienende Ammoniumgruppen sind beispielsweise zu nennen: Ammonium, N-Butylammonium, N-Cyclohexylammonium, N-Octadecylammonium, Trimethylammonium, Triethylammonium, Tributylammonium

Als 1- bis 4-wertige Metallionen kommen z.B. in Betracht:
Li, Na, K, Mg, Ca, Zn, Al, Ti.

Als Mercaptoalkylcarbonsäuren der obigen Formeln (IIa) und (IIb) kommen z.B. in Betracht Thioglykolsäure, 2-Mercaptopropionsäure, 3-Mercaptopropionsäure, 4-Mercaptobuttersäure, ω-Mercaptoundecansäure, 2-Mercaptobernsteinsäure, besonders bevorzugt 2-Mercaptopropionsäure, 3 -Mercaptopropionsäure und 2-Mercaptobernsteinsäure.

Als nach dem erfindungsgemäßen Verfahren bevorzugt hergestellte Polythiopolycarbonsäuren sind solche der nachfolgenden Formeln zu nennen:

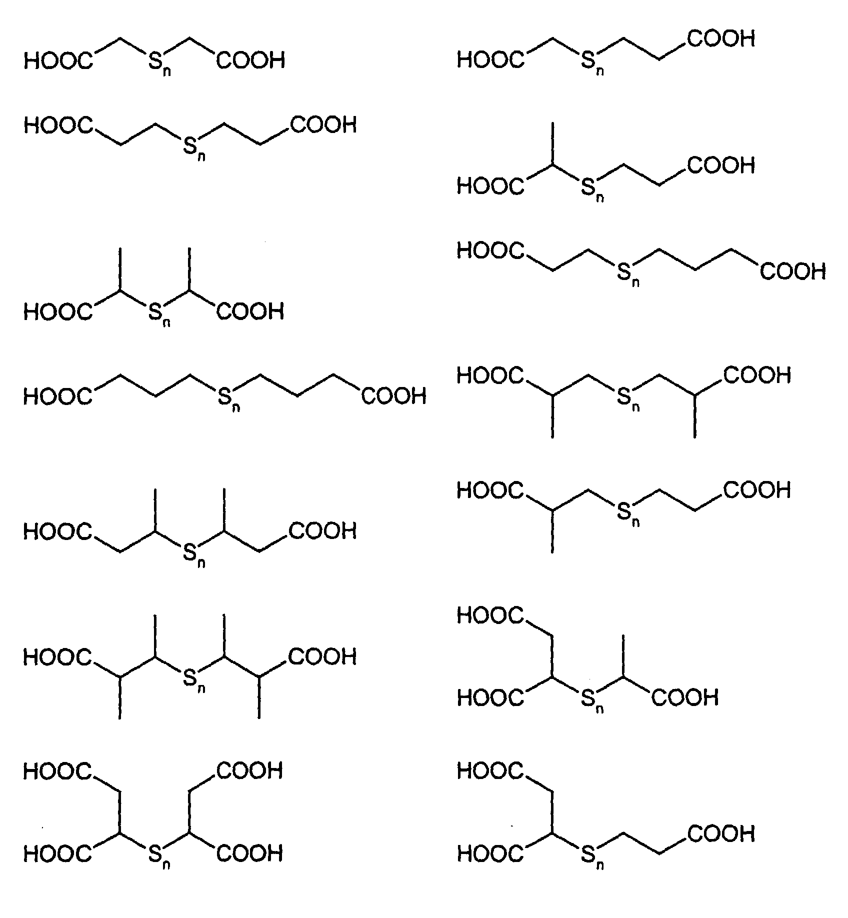

Einzubeziehen in die bevorzugten Verbindungen sind selbstverständlich deren entsprechenden Ammonium- oder Metallsalze. In den Formeln hat die Zahl n die zuvor genannte Bedeutung mit dem beschriebenen Vorzugsbereich.

Aufgrund des Einbaus unterschiedlicher Mengen an Schwefel während des erfindungsgemäßen Verfahrens handelt es sich bei den Verbindungen (1) in der Regel um Gemische von Polysulfidverbindungen, so dass n in der Bruttozusammensetzung der Verbindung (1) auch ungeradzahlige arithmetische Mittelwerte einnehmen kann, so dass der Mittelwert n ganz besonders bevorzugt im Bereich von 2,5 bis 7, insbesondere 2,5 bis 5 liegt.

Zur Durchführung des erfindungsgemäßen Verfahrens vermischt man die in Frage kommenden Mercaptocarbonsäuren entweder einzeln oder im Gemisch untereinander mit Schwefel im zuvor angegebenen Molverhältnis und erhitzt das Gemisch auf die ebenfalls angegebenen Temperaturen. Bei der Umsetzung entweicht Schwefelwasserstoff. Die Reaktionszeiten der Umsetzung liegen je nach Reaktionsbedingungen und eingesetzter Mercaptocarbonsäure im Bereich von wenigen Minuten bis zu mehreren Stunden.

Die erfindungsgemäße Umsetzung kann durch Katalysatoren beschleunigt werden. Geeignete Katalysatoren sind neben Wasser z.B. basische Verbindungen, wie Amine, z.B. Cyclohexylamin, primäre C₁₂- oder C₂₄-Alkylamine, wie Dodecylamin, Oleylamin oder Stearylamin, oder saure Verbindungen, wie Dischwefeldichlorid.

Die Katalysatoren werden bevorzugt in Mengen von 0,01 bis 1 Gew.-%, insbesondere 0,05 bis 0,5 Gew.-%, bezogen auf die Gesamtmenge des Reaktionsgemisches, eingesetzt.

Die erfindungsgemäße Umsetzung kann gegebenenfalls in einem geeigneten inerten Lösungsmittel durchgeführt werden, wie einem Ether, z.B. Tetrahydrofuran und Dioxan, oder einem aliphatischen Kohlenwasserstoff, wie Cyclohexan. Bevorzugt wird das erfindungsgemäße Verfahren jedoch ohne ein Lösungsmittel durchgeführt.

Nach der Umsetzung kann das Reaktionsgemisch zur weiteren Reinigung und Entfernung des gelösten Schwefelwasserstoffs mit bekannten Maßnahmen nachbehandelt werden, z.B. durch Entgasen im Vakuum oder Ausblasen mit Stickstoff oder Luft, Waschen mit Wasser oder Extraktion mit organischen Lösungsmitteln oder durch Behandlung mit entsprechenden Oxidationsmitteln, wie z.B. Wasserstoffperoxid oder organische Peroxide. Im Normalfall reicht die Reinheit der erhaltenen Polythiopolycarbonsäure nach dem Entgasen schon aus, so dass auf eine weitere Wäsche mit Wasser oder Extraktion mit organischen Lösungsmitteln verzichtet werden kann.

Zur Herstellung der erfindungsgemäßen Kautschukmischungen mit den erfindungsgemäß hergestellten Polythiocarbonsäuren eignen sich sowohl Kautschuk als auch Synthesekautschuke.

Bevorzugte Synthesekautschuke sind beispielsweise bei W. Hofmann, Kautschuktechnologie, Genter Verlag, Stuttgart 180 und I. Franta, Elastomers und Rubber Compounding Materials, Elsevier, Amsterdam 1989 beschrieben. Sie umfassen u.a.
- BR: Polybutadien
- ABR: Butadien/Acrylsäure-C₁₋₄-alkylester-Copolymere
- CR: Polychloropren
- IR: Polyisopren
- SBR: Styrol/Butadien-Copolymerisate mit Styrolgehalten von 1-60, vorzugsweise 20-50 Gew.-%
- IIR: Isobutylen/Isopren-Copolymerisate mit Isoprengehalten von 0,01 bis 5 Gew.-% (Butylkautschuk)
- BIR-IIR: bromierte Isobutylen-Copolymerisate mit Bromgehalten zwischen 0,01 und 4 Gew.-% (Brombutylkautschuk)
- CI-IIR: Chlorierte Isobutylen-Copolymerisate mit Chlorgehalten zwischen 0,01 und 4 Gew.-% (Chlorbutylkautschuk)
- BIMS: bromierte Copolymerisate mit 0,05 bis 3 Mol.-% benzylisch gebundenem Brom
- NBR: Butadien/Acrylnitril-Copolymere mit Acrylnitrilgehalten von 5-60, vorzugsweise 10-40 Gew.-%
- HNBR: teilhydrierter oder vollständig hydrierter NBR-Kautschuk
- EPDM: Ethylen/Propylen/dien-Copolymerisate, mit Diengehalten zwischen 0,1 bis 20 Gew.-%, vorzugsweise 0,5 bis 10 Gew.-%
- CO: Polyepichlorhydrin
- ECO: Copolymere von Epichlorhydrin und Ethylenoxid
- EAM: Ethylen/Vinylacetat-Copolymere mit Vinylacetatgehalten von 20 bis 90 Gew.-%, vorzugsweise 40 bis 80 Gew.-%
sowie Mischungen dieser Kautschuke. Für die Herstellung von Kfz-Reifen sind insbesondere Naturkautschuk, Polyisopren, Emulsions-SBR sowie Lösungs-SBR-Kautschuke mit einer Glastemperatur oberhalb von -50°C, die gegebenenfalls mit Silylethern oder anderen funktionellen Gruppen nach EP-A 447 066 modifiziert sein können, Polybutadienkautschuk mit hohem 1,4-cis-Gehalt (>90 %), der mit Katalysatoren auf Basis Ni, Co, Ti oder Nd hergestellt wurde, sowie Polybutadienkautschuk mit einem Vinylgehalt von bis zu 75 %, Butylkautschuk, Brombutylkautschuk, Chlorbutylkautschuk sowie deren Mischungen von Interesse.

Als Füllstoffe kommen für die erfindungsgemäßen Kautschukmischungen alle bekannten in der Kautschukindustrie verwendeten Füllstoffe in Betracht, diese umfassend sowohl aktive als auch inaktive Füllstoffe.

Zu erwähnen sind:
- hochdisperse Kieselsäuren, hergestellt z.B. durch Fällung von Lösungen von Silikaten oder Flammhydrolyse von Siliciumhalogeniden mit spezifischen Oberflächen von 5 bis 1 000, vorzugsweise 20 bis 400 m²/g (BET-Oberfläche) und mit Primärteilchengrößen von 10 bis 400 nm. Die Kieselsäuren können gegebenenfalls auch als Mischoxide mit anderen Metalloxiden, wie Al-, Mg-, Ca-, Ba-, Zn-, Zr-, Ti-ociden vorliegen;
- synthetische Silikate, wie Aluminiumsilikat, Erdalkalisilikat wie Magnesiumsilikat oder Calciumsilikat, mit BET-Oberflächen von 20 bis 400 m²/g und Primärteilchendurchmessern von 10 bis 400 nm;
- natürliche Silikate, wie Kaolin und andere natürlich vorkommende Kieselsäure;
- Glasfasern und Glasfaserprodukte (Matten, Stränge) oder Mikroglaskugeln;
- Metalloxide, wie Zinkoxid, Calciumoxid, Magnesiumoxid, Aluminiumoxid;
- Metallcarbonate, wie Magnesiumcarbonat, Calciumcarbonat, Zinkcarbonat;
- Metallhydroxide, wie z.B. Aluminiumhydroxid, Magnesiumhydroxid;
- Ruße
   Die hierbei zu verwendenden Russe sind nach dem Flammruß, Furnace- oder Gasrußverfahren hergestellt und besitzen BET-Oberflächen von 20 bis 200 m²/g, z.B. SAF-, ISAG-, HAF-, FEF- oder GPF-Ruße;

Bevorzugt werden als Füllstoffe hochdisperse Kieselsäuren und/oder Ruße eingesetzt, wobei das Mischungsverhältnis von Ruß zu Kieselsäure bei 0,05 bis 30, besonders bevorzugt bei 0,1 bis 10 liegt.

Die erfindungsgemäßen Kautschukmischungen können darüber hinaus noch andere Kautschukhilfsmittel enthalten, die beispielsweise der weiteren Vernetzung der Kautschukmischungen dienen, oder die die physikalischen Eigenschaften der aus den erfindungsgemäßen Kautschukmischungen hergestellten Vulkanisate für deren speziellen Einsatzzweck verbessern.

Als Vernetzeragentien werden Schwefel oder Schwefel-liefernde Verbindungen oder Peroxide eingesetzt. Besonders bevorzugt werden Schwefel oder Schwefel-liefernde Verbindungen in Mengen von 0,01 bis 3 Gew.-Teile bezogen auf Kautschuk. Darüber hinaus können, wie erwähnt, die erfindungsgemäßen Kautschukmischungen weitere Hilfsmittel, wie die bekannten Reaktionsbeschleuniger, Alterungsschutzmittel, Wärmestabilisatoren, Lichtschutzmittel, Ozonschutzmittel, Verarbeitungshilfsmittel, Verstärkerharze, z. B. Phenolharze, Stahlcord-Haftmittel, wie z. B. Kieselsäure/Resorcin/Hexamethylentetramin oder Cobalt-Naphthenat, Weichmacher, Tackifier,Treibmittel, Farbstoffe, Pigmente, Wachse, Streckmittel, organische Säuren, Verzögerer, Metalloxide sowie Füllstoffaktivatoren, insbesondere polysulfidische Silane, wie Bis-(triethoxisilylpropyl)-tetrasulfid, enthalten.

Die erfindungsgemäßen Kautschukhilfsmittel werden in den üblichen, bekannten Mengen eingesetzt, wobei sich die eingesetzte Menge nach dem späteren Verwendungszweck der Kautschukmischungen richtet. Üblich sind beispielsweise Mengen an Kautschukhilfsmitteln im Bereich von 2 bis 70 Gew.-Teilen, bezogen auf 100 Gew.-Teile Kautschuk.

Die erfindungsgemäßen Kautschukmischungen können z. B. hergesellt werden durch Abmischung der Kautschuke mit den erfindungsgemäß hergestellten Polythiocarbonsäuren, Füllstoffen, Kautschukhilfsmitteln und Vernetzern in geeigneten Mischapparaturen, wie Knetern, Walzen oder Extrudern.

### Beispiel

### Herstellung von HOOC-CH₂CH₂-Sₙ-CH₂CH₂-COOH

Ein Gemisch aus 53 g (0,5 mol) 3-Mercaptopropionsäure, 24 g (0,75 mol) Schwefel sowie 0,3 g Oleylamin wurde auf 130°C erhitzt, wobei Schwefelwasserstoff-Entwicklung einsetzte. Es wurde 2,5 Stunden bei 130°C nachgerührt und anschließend 5 Minuten Vakuum angelegt, um restlichen Schwefelwasserstoff zu entfernen. Nach dem Abkühlen wurden 69,1 g Polythiodipropionsäure als hellgelbe Kristalle erhalten. Schmelzbereich: 95 bis 130°C. Das HPLC-Chromatogramm zeigt, dass es sich um ein Gemisch homologer Polysulfide handelt mit n = 3-11. (Mittelwert: 4).

| Elementaranalyse: | C | H | S |
|---|---|---|---|
| Berechnet für -S₄- | 26,3 | 3,7 | 46,7 % |
| Gefunden | 26,3 | 3,6 | 47,5 % |

## Patentansprüche

1. Verfahren zur Herstellung von Polythiopolycarbonsäure der Formel
R¹-(S)ₙ-R² (I),
worin
R¹ und R² gleich oder verschieden sind und für einen linearen oder verzweigten C₁-C₃₆-Alkylrest, oder einen C₅-C₁₈-Cycloalkylrest stehen, wobei der Alkylrest und der Cycloalkylrest mit 1 bis 4 Carboxyl- oder Carboxylatgruppen substituiert sind und wobei im Falle einer Carboxylatgruppe als Kation eine Ammoniumgruppe, eine C₁-C₁₈-Mono-, Di- oder Trialkylammoniumgruppe, eine C₆-C₁₈-Mono-, Di- oder Triarylammoniumgruppe, eine C₇-C₂₁-Mono-, Di- oder Trialkylarylammoniumgruppe oder ein 1- bis 4-wertiges Metallion vorliegt, und
n für eine ganze Zahl von 2 bis 14 steht,
dadurch gekennzeichnet, dass man Mercaptoalkylcarbonsäuren der Formeln
R¹-S-H (IIa),
R²-S-H (IIb),
worin
R¹ und R² die obige Bedeutung besitzen,
oder Mischungen entsprechender Mercaptoalkylcarbonsäuren mit Schwefel im Molverhältnis von 1 zu 0,25 bis 5 bei Temperaturen von 70 bis 220°C, gegebenenfalls in Gegenwart von Katalysatoren und gegebenenfalls in Anwesenheit eines inerten Lösungsmittels umsetzt.

2. Verwendung der gemäß dem Verfahren nach Anspruch 1 hergestellten Polythiopolycarbonsäuren zur Herstellung von Kautschukmischungen und Kautschukvulkanisaten.
